# EUROPEAN PATENT APPLICATION

(11) **EP 3 756 620 A1**
(43) Date of publication of application: **30.12.2020**
(21) Application number: 20380016.4
(22) Date of filing: 11.06.2020
(51) Int. Cl.: A61F 2/07

(54) **MULTICHANNEL STENT WITH PREASSEMBLED GUIDE SYSTEMS**

(30) Priority: 14.06.2019 ES 201900093 U
(71) Applicant: Escribano Uzcudun, Miguel, 20011 San Sebastian (ES)
(72) Inventor: Sanchez Abuin, Javier, 20014 San Sébastián (ES); Escribano Uzcudun, Miguel, 20011 San Sébastián (ES); Mikelarena Monteiro, Ederi, 20018 San Sébastián (ES)

(57) **Abstract**

Aortic aneurysms are currently treated with aortic stents in a very high percentage of cases.

There are currently multiple models and patents for stents.

The stent that we have invented is an innovative platform that solves most of the problems presented by the stents used in the treatment of aneurysms that affect the visceral aorta.

This new endoprosthesis is characterized by having several channels of different sizes inside to allow the passage of blood or other physiological fluid through them, but not between them or outside. These interior passages or channels are arranged such that they have an input and output on the longitudinal axis and do not have inputs or outputs [20] on the sides of the stent. They are also of equal length; thus their origin and their end are at the same height as the longitudinal axis of the stent, and these passages or channels do not communicate with each other.

These interior passages or channels consist of tubular structures of biocompatible material, that are supported throughout its entire length, or in part, by stents similar to those of the external body, which are similar to the stents currently available.

## Description

A new stent characterized by having various channels of different sizes inside to allow the passage of blood or other physiological fluid through them, but not between them.

These interior passages or channels consist of tubular structures of biocompatible material that are supported throughout its entire length, or in part, by stents similar to those of the external body, which is itself similar to stents that are currently available on the market.

Unlike other stents currently available, however, this new stent has several channels inside of different sizes to allow the passage of blood or other physiological fluid through them, but not between them or outside. These interior passages or channels are arranged such that they have an inlet and outlet on the longitudinal axis and do not have inlets or outlets on the sides of the stent. They are also of equal length; thus their origin and their end is at the same height as the longitudinal axis of the stent, and the passages or channels do not communicate with each other.

### TECHNOLOGICAL SECTOR

The technological sector in which the invention falls is within the field of medical-surgical implants, specifically those known as endoprosthesis, stents, stent grafts, and also more recently scaffolds.

### BACKGROUND OF THE INVENTION

Aortic aneurysms are currently treated with aortic stents in a very high percentage of cases.

There are multiple stent models and patents. However, so far no endoprosthesis has been developed that allows for the treatment of the visceral aorta and that in turn avoids the problems presented by the current ones:
h) Design and measurement complexity in choosing and making the correct stent adapted to each patient.
i) Lack of versatility to deal with multiple cases with few standardized models.
j) Lack of immediate availability. Most cases require the use of a custom stent, so this makes it difficult to store stents in hospitals for immediate use.

Thus, an endoprosthesis designed with few components is needed, which does not require particularly complex measurements to enable its implant, and which in turn can be stored in hospitals. This will allow for both immediate availability and adaptation to most patients. Furthermore, if it also uses a design that facilitates the implantation process, this makes it especially useful in cases where there is a life-threatening emergency, given its accessibility, design advantages and the simplification of implant steps.

The prosthesis that we have invented is a radically different and absolutely innovative platform presenting better properties and greater capacities compared to current prostheses.

Our stent also meets the following requirements:
a) Greater simplicity when choosing the right model.
b) Versatility to treat most patients with a few standardized models (and therefore easy to store and manufacture).
c) Simplification of the stent implant.

### EXPLANATION OF THE INVENTION

The invention consists of a stent composed of multiple self-expanding metal stents, joined by a layer of biocompatible material, like others currently on the market.

Unlike the rest of the stents currently available, however, this new stent has several passages of different sizes inside to allow the passage of blood (or other physiological fluid) through them, but not between or outside of them. These interior passages or channels are arranged such that they have an inlet and outlet on the longitudinal axis and do not have inlets or outlets on the sides of the stent. They are also of equal length; thus their origin and their end are at the same height as the longitudinal axis of the stent, and these passages or channels do not communicate with each other.

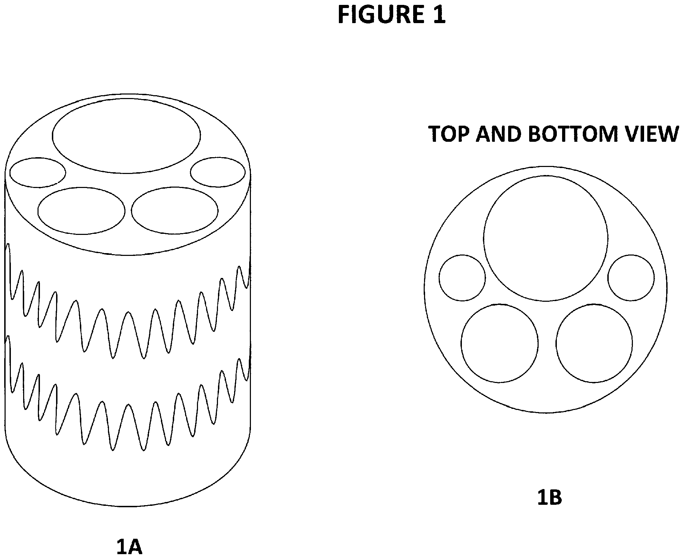

These interior passages or channels consist of tubular structures of the same or similar biocompatible material, and supported throughout their entire length, or in part, by the same or similar stents, although smaller in size than those of the external body. At both ends, the biocompatible material covers the spaces between the different passages so that biological liquids such as blood only pass through the stent within the interior passages, and do not pass through the spaces between them. This produces a tube with several channels inside. By way of example we will describe one of the most common configurations of the stent, which is one with five channels inside, with one of them being larger *(**Figures 1A and 1B**).*

Continuing with the same example, for its implant it will form a system such that:
What we will refer to from now as the "primary channel" (which will normally have the largest diameter) is traversed by the guide that is used to lead the stent to the desired place (as in current standard procedures). We will call this guide, which is independent of the prosthesis and its introduction and release system, the "external guide."

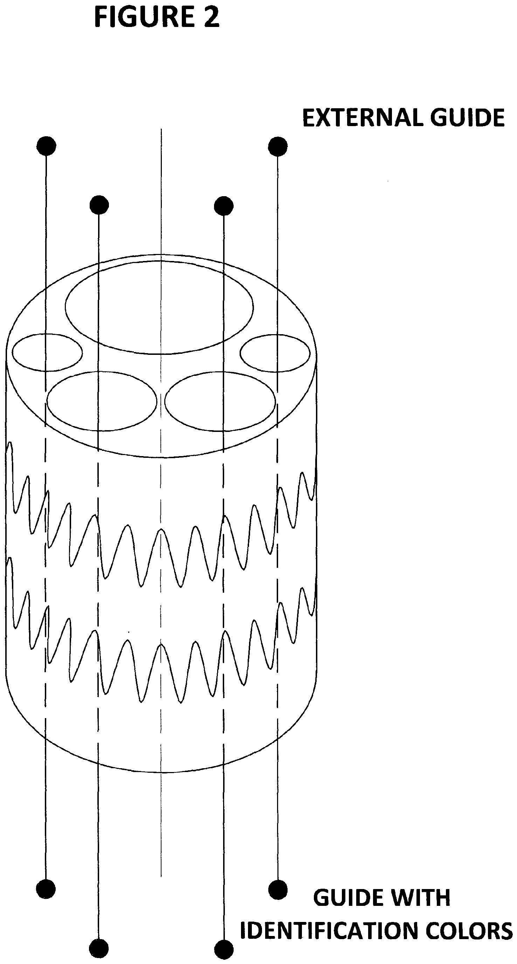

In the example that we have used (although this need not necessarily be the case), other guides (preferably smaller in diameter than the external guide) are incorporated into the prosthesis release system and are part of it. Henceforth, we will call them "internal guides," with each of them traversing through the other channels of the stent, which we will call "accessory channels." These accessory channels range from 1, 2, 3, 4 or up to 5 in number. *Figure 2* shows the prosthesis that we have just described with the system of guides in its interior, in such a way that the external guide passes through the primary channel with the largest diameter and the other guides pass through the accessory channels.

Like all current stents, ours will be folded and inserted into a system for its introduction and release inside the human body. For brevity, we will call this system the "release system." Each of the internal guides is visible (and therefore allows its use) from the exterior of the patient through the release system, in such a way that each one will have a distinctive indicator (for example, colors) to identify the accessory channel that they traverse.

The stent will also have different radiopaque markers to guide its release, as is done with the stents already on the market.

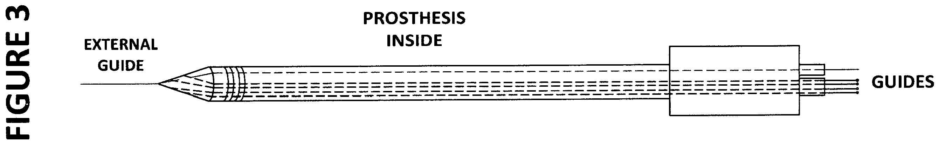

The following illustration *(**Figure 3**)* shows the outline of the prosthesis release system channeled through the external guide, as well as the guides that pass through the accessory channels. As can be seen, these guides will have two ends: the end that does not penetrate the patient's body and that allows its handling from the access site; and the end that will be attached to the tip of the introduction system and fixed to it.

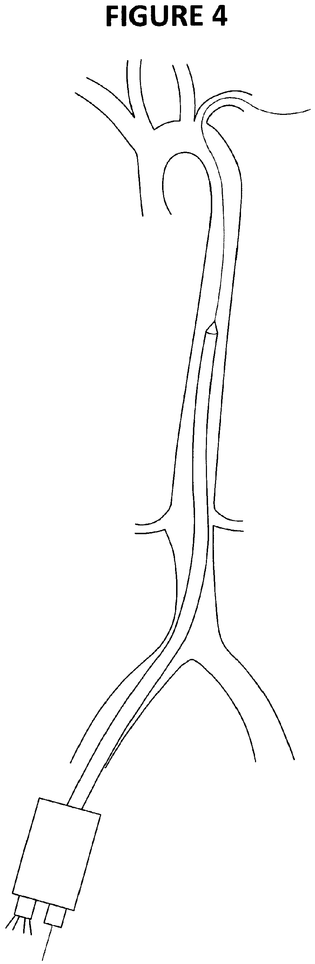

Once the delivery system is inserted into the body through the external guide (generally from the common femoral artery, but not exclusively), this external guide can be captured from an arm-level access (or other arterial access, which we will call "secondary access") as is done in standard procedures.
The following illustration from our example shows the progression of the introducer system through the external guide to the interior of the aorta *(**Figure 4**).*

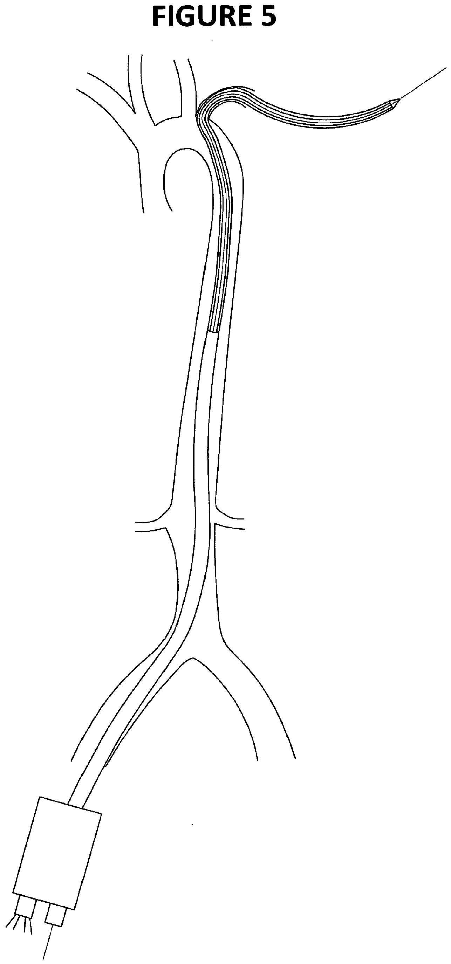

Once this guide has been captured and extracted through the arm (secondary access), the device will allow the tip or a distal segment (1) to be removed from the stent delivery system through the secondary access. This segment incorporates the other end of the internal guides, and in this way they can be introduced through the introducer of the secondary access (for example, the axillary artery) and exposed outside the patient's body, as shown in the illustration *(**Figure 5**).*

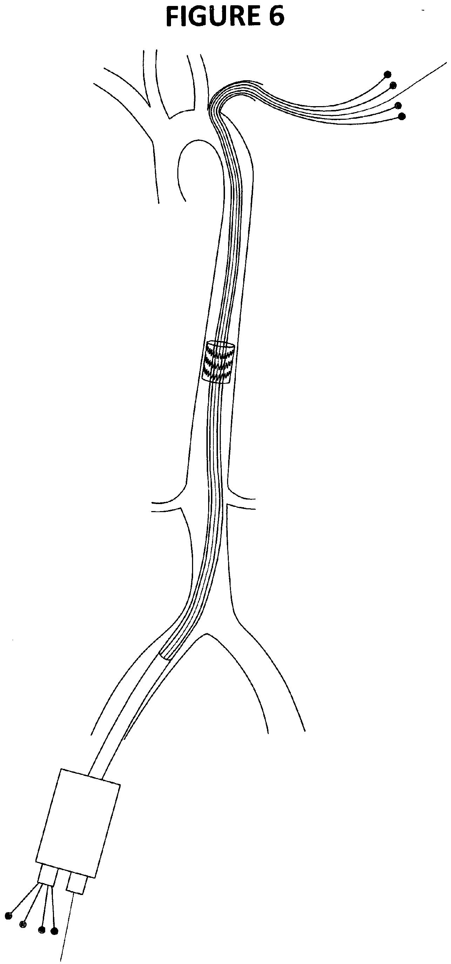

At this point in the procedure we can release the stent we are describing in our example, inside the body. We would thus have the outer guide through the primary channel and the inner guides through each secondary channel: both ends of each guide are exposed on the outside of the patient, both by the initial access and by the secondary access. The identification codes of each secondary guide allow us to know through which secondary channel it has its trajectory. The following image *(**Figure* 6) shows the result of the steps after prosthesis release. The prosthesis is released from the delivery system, which can be done in the same way as in most commercially available stents: removing the introducer sheath of the delivery system causing the stent to expand inside the body, which in our example would be into the supravisceral aorta. In this way, we have the expanded prosthesis with each passage of the stent channeled with a guide, and each of the guides controlled in both arterial accesses (primary and secondary) *(**Figure 6**).*

This arrangement allows us, through the primary channel and with the external guide, and through the secondary channel with the internal guides, to use other stents similar to those already on the market today. These conventional stents allow us to connect our stent to the iliac arteries and other main branches of the aortic artery, thus allowing for the passage of blood to them as in a conventional procedure.

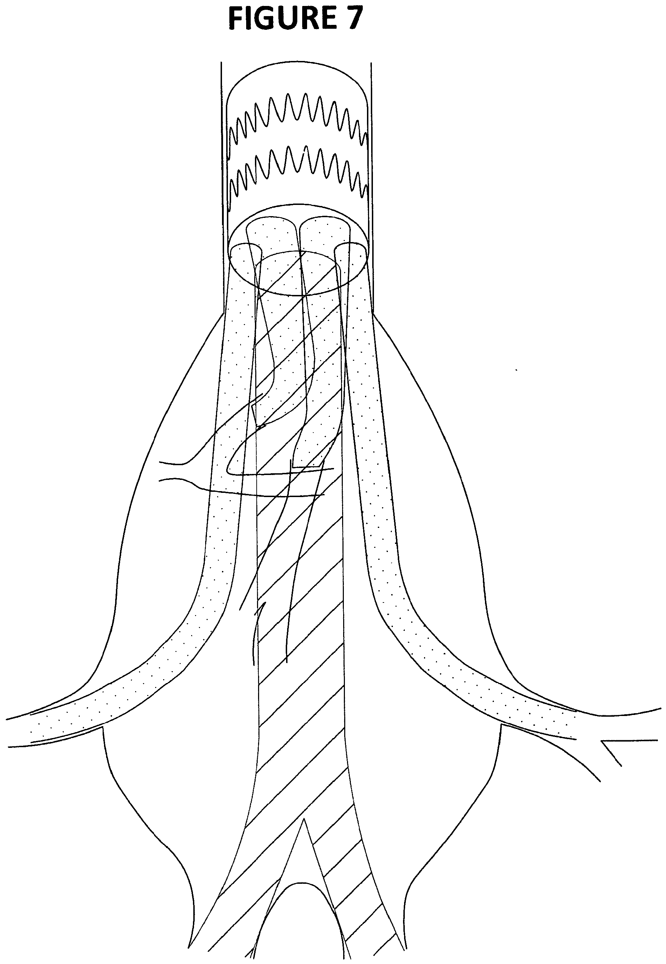

Next, a diagram is shown where our stent can be seen already unfolded together with the different conventional stents that connect with the different arterial branches and, in this example, excludes the aneurysm to be treated *(**Figure 7**).*

Our example shows that our stent is completely different from current models:
Document US6942692B2 "Adrenal Prosthesis and Renal Artery Bypass" consists of a first stent or stent joint with sealing material and other prostheses to bypass the aneurysm and with the possibility of a prosthesis for a renal artery. The composition, arrangement of the prosthesis and the aneurysm sealing system are all very different from ours, and because of this, it does not allow for the treatment of the entire visceral aorta.

Document ES2589303T3 "Dual-Channel Aortic Arch Main Body Stent and Methods of Use." It is a stent with a main body with a bifurcation that defines two "legs," and its diameter can vary. It has common elements with commercially available stents but it has a totally different arrangement from ours. This prosthesis has "legs," while ours has internal channels. A great advantage of the internal channels is that they are located in the sealing zone (unlike the "legs" of this stent). This means a shorter distance between the endoprosthesis release zone and the visceral arteries, with the advantage of covering less aorta which, in turn, reduces the risk of fatal complications such as paraplegia. In addition, the different braces with different number of channels give our prosthesis more versatility to adapt to a greater number of patients.

Another advantage of the design in our example is that it allows stent systems (or covered stents) to be advanced to the different visceral arteries with greater comfort and support, thanks to the guides that can traverse through the accessory channels, thus simplifying the procedure to a great extent with respect to current stents.

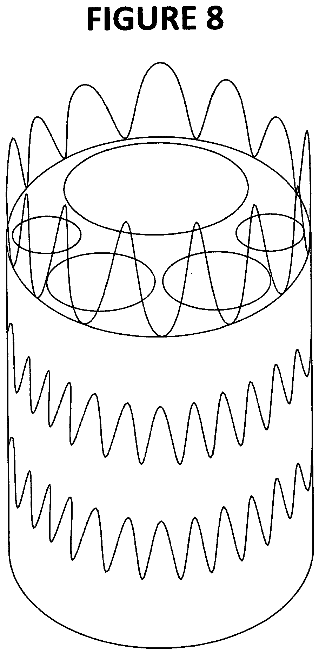

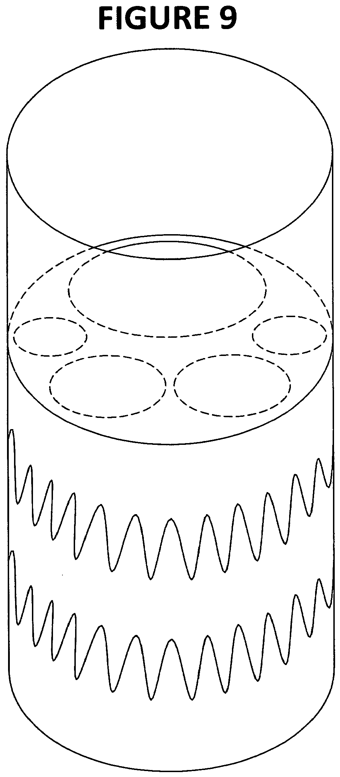

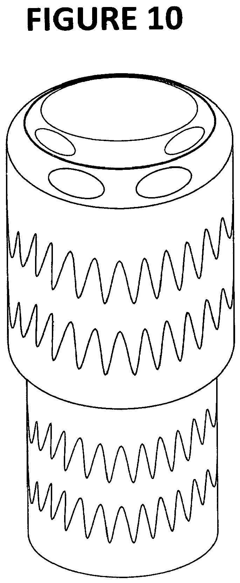

Apart from the basic elements of the design, such as those described in the example, our system allows for multiple variants to improve its function. Without being exhaustive or exclusive, these are:
- It allows for the incorporation of a proximal stent free of coverage (with or without hooks), to increase fixation to the arterial walls. The illustration represents a possible configuration of our stent, with a proximal free stent to improve its fixation *(**Figure 8**).*
- It allows for the incorporation of one or more proximal stents covered with biocompatible tissue, creating a first segment without channels inside, with the internal channels being in a second segment of the stent *(**Figure 9**)*. This variant seeks to optimize the sealing of the stent and/or guarantee the overlap with other possible stents. This design alternative thus has the advantage of being able to improve fixation by means of systems such as EndoAnchors or Aptus (Heli-FX system, already commercialized) that fix the endoprosthesis to the aortic wall or to a more proximal prosthesis. It can also facilitate a possible proximal extension of the stent, maintaining the sealing of the aneurysm, in the event that this added treatment is needed in the future.
- The two previous descriptions are not exclusive, and a stent can be manufactured with a multichannel segment, a conventional coated tubular segment and a segment with uncoated proximal fixation stent.
- Another possible configuration would be a design such that the proximal part of the stents that originate the different channels and the biocompatible tissue between them, has an oblique morphology (describing a descending obliqueness from the external part of the prosthesis to the internal part). In this way, we could generate a more favorable hemodynamic behavior of the blood towards the different channels of the stent.
- The different combinations (with a different number of channels inside) allow for greater versatility of the device. A stent containing two parallel channels inside can also be considered where, in one of the two channels, a second multi-channel stent is inserted where all the channels are the same dimension to treat the visceral branches and the other channel is extended to treat the aorta.
- Another variant can be imagined that would have the ability to decrease the necessary length of the covered stents (or stents) that go to the visceral branches. This variant would consist of a first stent with one primary channel and two accessory channels. These accessory channels would be used to funnel the most proximal visceral branches. Inside the primary channel, another stent of smaller diameter and greater length can be placed with two accessory channels. Through these other accessory channels the most distal visceral arteries can be funneled and extend through the primary channel to the aorta, as shown in the example in *Figure 10**.*

With a small number of prostheses of different diameters for the main body (for example, and without being exhaustive or exclusive, of 24, 28, 32, 36, 40 mm) and different lengths (for example, 30, 40, 50 cm), together with the different accessory channel designs, our device and its different variants allow for the treatment of most aneurysms that affect the visceral arteries.

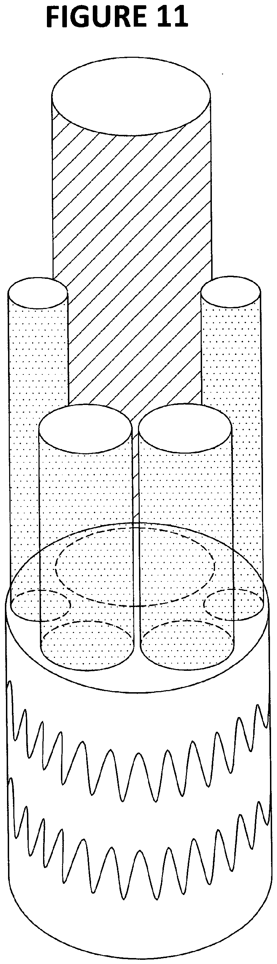

It should also be noted that our device allows for modifications in its implant and in the form of release previously described in our example, such as channeling of the visceral arteries (and also the supra-aortic arteries) in a retrograde manner. The following illustration shows by way of example the schematic of an implant, where the stents connecting to the different branches are placed in a retrograde arrangement *(**Figure 11**).*

This possibility allows its use to be extended for the treatment of more patients, particularly for cases with complex anatomies that pose a challenge and are currently difficult to treat.

The device would also allow for the modification of the release procedure (for example, releasing the prosthesis before capturing the accessory guides), thus being able to facilitate the implantation process in certain cases. Another characteristic would be the ability to remove the internal guides from the initial or primary access without the need to extract the other end through the secondary access.

We can therefore conclude that our design - with its different variants - allows us to meet the stated objectives of ease of use and versatility extending its application to many more patients.

### PREFERRED APPLICATION OF THE INVENTION

It is evident that this invention is capable of immediate application in the medical-surgical implant industry.

### BRIEF EXPLANATION OF THE DRAWINGS

Drawing 1
   It shows one of the most common configurations of the stent, with five channels inside, one of them larger.
Drawing 2
   It shows the stent shown in the previous diagram (Drawing 1) but with the guides inside: the external guide through the larger diameter channel (primary) and the other guides that traverse the accessory channels.
Drawing 3
   It shows a diagram of what the introducer system of the stent would be like inside the body, already channeled with the external guide. The stent is housed inside the introducer system and the external guide passes through the primary channel. The drawing also shows the guides that pass through the accessory channels and are connected to the tip of the introducer (1) and free at the other end of the introducer.
Drawing 4
   It shows the introducer system represented in Drawing 3 but already inside the aorta, and having progressed in it via the external guide.
Drawing 5
   It shows one of the steps of the implant of the stent, specifically the model which has the accessory channel guides (as shown in the previous drawings). In this way, the external guide is captured and taken out of the body through a secondary access (axillary artery in the drawing). Through this access, the tip of the introducer system (1) that contains one end of each of the guides that traverse the accessory channels is externalized.
Drawing 6
   The following steps are represented in the release of the stent described in Drawing 5. The tip of the introducer is removed on the outside of the body and the other end of the guides that go through the accessory channels are captured. The stent is released from the introducer system causing it to expand inside the body. Thus we have the expanded stent, with each passage of the stent channeled with a guide and each of the guides controlled in both arterial accesses (primary and secondary).
Drawing 7
   It shows a diagram of the stent already released and the stents that connect each channel to the different arterial branches already deployed.
Drawing 8
   It is the image of another configuration of the stent with a proximal free stent to improve its fixation to the arterial walls.
Drawing 9
   It is the image of another configuration of the stent but this time with a proximal coated stent, as explained in the text, that may have its advantages for fixation or overlap with other stents.
Drawing 10
   It shows another configuration with three channels, presenting different diameters that can give more options for use and be combined among them.
Drawing 11
   It is the image of an implant, but carried out with a different configuration: it presents the connecting stents with the different branches placed in a retrograde arrangement.

## Claims

1. Endoprosthesis composed of multiple self-expanding metal stents joined by a layer of biocompatible material **characterized by** having several passages or channels inside of different sizes, which allow blood or other biological liquid to pass only through the interior of these channels and not outside or between them.
These interior passages or channels are arranged such that they have an input and outlet on the longitudinal axis and do not have inputs or outlets on the sides of the stent. They are also of equal length; thus their origin and their end are at the same height as the longitudinal axis of the stent, and these passages or channels do not communicate with each other.
These channels and the external body are made up of similar but different stents of different size, and joined by the same or similar biocompatible tissue.

2. The stent of Claim 1 is **characterized by** its proximal area having a first segment formed by one or more support stents covered by biocompatible tissue, forming a single proximal channel in said segment.

3. The stent of Claims 1 and 2 is **characterized by** its proximal end having a free stent not covered by self-expanding biocompatible tissue to increase its fixation.

4. The stent of Claims 1, 2 and 3 has the characteristic that once it is included in the introducer system it has all the passages except one already channeled by guides included in the introducer system which can be removed from both ends of the introducer system. The remaining passage is channeled by a guide not included in the introducer system, as in traditional devices.
